(19) Europäisches Patentamt
European Patent Office
Office européen des brevets

(11) **EP 3 142 540 B1**

(12) **EUROPEAN PATENT SPECIFICATION**

(45) Date of publication and mention
of the grant of the patent:
**19.01.2022 Bulletin 2022/03**

(51) International Patent Classification (IPC):
**A61B 1/06** (2006.01)

(21) Application number: **15727456.4**

(52) Cooperative Patent Classification (CPC):
**A61B 1/303; A61B 1/0607; A61B 1/0615**

(22) Date of filing: **17.04.2015**

(86) International application number:
**PCT/IB2015/052808**

(87) International publication number:
**WO 2015/173676 (19.11.2015 Gazette 2015/46)**

(54) **COLPOSCOPY APPARATUS FOR PERFORMING A COLPOSCOPY PROCEDURE**

KOLPOSKOPIEVORRICHTUNG ZUR DURCHFÜHRUNG EINES KOLPOSKOPIEVERFAHRENS

APPAREIL DE COLPOSCOPIE POUR MISE EN OEUVRE D'UNE PROCÉDURE DE COLPOSCOPIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.05.2014 US 201461992974 P**

(43) Date of publication of application:
**22.03.2017 Bulletin 2017/12**

(73) Proprietor: **Koninklijke Philips N.V.
5656 AG Eindhoven (NL)**

(72) Inventors:
• **SETH, Subhendu
NL-5656 AE Eindhoven (NL)**
• **SOLANKI, Chirag
NL-5656 AE Eindhoven (NL)**
• **VAJINEPALLI, Pallavi
NL-5656 AE Eindhoven (NL)**

(74) Representative: **Philips Intellectual Property &
Standards
High Tech Campus 52
5656 AG Eindhoven (NL)**

(56) References cited:
**WO-A1-2014/006549     US-A1- 2006 215 406
US-A1- 2010 025 566     US-A1- 2012 249 764
US-B1- 6 277 067**

**Description**

FIELD OF THE INVENTION

The present invention relates to the medical field and, in particular, relates to a colposcopy apparatus for performing a colposcopy procedure.

BACKGROUND OF THE INVENTION

Cervical cancer is the second most common cancer in women. In order to visually check the surface of the cervix for abnormalities, a colposcopy procedure is performed, wherein a device called a vaginal speculum is inserted into the vagina and the cervix is examined by a gynecologist using an instrument called a colposcope having a light and a magnifying lens, which stays outside the body and allows the gynecologist to see any abnormalities. As part of the colposcopy procedure, the gynecologist may initially clean the cervix with normal saline to remove any secretions. This may be followed by looking at the cervix under a green filter for determining the presence of blood vessels and other vascular patterns like punctuations and mosaics. Thereafter, a further step of applying a diluted acetic acid which turns the color of the abnormal cervical tissue to white may follow, wherein the gynecologist maps the time of appearance of this Aceto-while effect and the duration for which the Aceto-whiteness stays. After this step, the gynecologist may further apply 5 % Lugol's iodine and check whether the cervical tissue shows a partial or complete uptake of the iodine.

To focus the colposcope and, thus, to obtain sharp images when performing the colposcopy procedure, the region of interest, here, the cervix, must be at the correct focal distance (i.e., focal length) from the colposcope. This focal distance, which corresponds to the working distance, is typically between 20 cm to 30 cm long. Shorter focal distances require the colposcope to be closer to the cervix, making it difficult to use other clinical instruments like a cotton swab, stains, therapy devices and the like when performing the colposcopy procedure. Longer focal distances, one the other hand, can make it uncomfortable for the gynecologist to perform the colposcopy procedure, in particular, if he/she has comparably short arms. In the clinical practice, positioning the colposcope at the proper working distance can be quite difficult due to the anatomy of the vagina as well as the slight movements of the cervix. Moreover, focus may be lost during a longer colposcopy procedure due to movements of the patient.

US 2006/0215406 discloses a colposcope for examining cervical tissue. WO 2014/006549 discloses a system for imaging a patient's interior. US 2010/0025566 discloses a system for the acquisition of high-quality focused tissue image data. US 2012/0249764 discloses a cervical, fetal-membrane, and amniotic examination and assessment device.

US 6,277,067 B1 discloses a hand-held colposcopy assembly capable of producing a digital image of the cervix. The colposcopy assembly comprises first and second light emitters which are able to generate emitted light beams visible to the human eye when seen against human tissue. The first and second light emitters are located on opposite sides of a fixed focus objective lens through which a CCD sensor based camera (charge-coupled device) can capture the digital image. Moreover, the first and second light emitters are oriented such that the emitted light beams intersect on the optical axis of the objective lens at the optimum focal plane of the camera, whereby, as the colposcopy assembly is aimed at cervical tissue of the patient, the emitted light beams produce first and second light spots on the tissue, the first and second light spots being observed by the gynecologist as a single light spot when the cervix is properly focused in the camera.

The configuration with the first and second light emitters supports a gynecologist in positioning the disclosed colposcopy assembly at the proper working distance from the cervix. However, it would also be helpful for the gynecologist if during the process of positioning the colposcope, he/she would additionally be supported in finding a position and/or configuration of the colposcope that allows seeing a maximum amount of the cervix. This problem is not addressed in the cited prior art.

SUMMARY OF THE INVENTION

It is therefore an object of the present invention to provide a colposcopy apparatus for performing a colposcopy procedure, which allows better supporting a user, for instance, a gynecologist, in positioning and/or configuring the colposcopy apparatus for performing the colposcopy procedure. The present invention is defined in and by the appended claims.

There is also described herein a colposcopy apparatus for performing a colposcopy procedure, wherein the colposcopy apparatus comprises:

- an imaging unit comprising an optics unit having an optical axis and an image acquiring unit for acquiring an image of a cervix through the optics unit, and
- at least three light beam emitting units spaced apart from the optical axis, wherein the at least three light beam emitting units are adapted to emit light beams that are coplanar with the optical axis and that intersect on the optical axis at a predefined distance in front of the colposcopy apparatus, wherein not all of the emitted light beams lie in a same plane.

Since the colposcopy apparatus comprises at least three light beam emitting units spaced apart from the optical axis, and since the at least three light beam emitting units are adapted to emit light beams that are coplanar with the optical axis and that intersect on the optical axis at a predefined distance in front of the colposcopy apparatus, wherein not all of the emitted light beams lie in a same plane, when the colposcopy apparatus is aimed at cervical tissue during the process of positioning the colposcopy apparatus, the emitted light beams produce a two-dimensional pattern of light spots on the tissue. The overall dimension of this two-dimensional pattern of light spots becomes smaller - ultimately converging into a single observable light spot - when the distance of the colposcopy apparatus from the cervix approaches the predefined distance, that is, the distance at which the emitted light beams intersect on the optical axis in front of the colposcopy apparatus, making it easy to position the colposcopy apparatus at the predefined distance from the cervix. In addition, when the orientation of the colposcopy apparatus changes with respect to the cervix, the shape of the two-dimensional pattern of light spots, that is, the relative distances between the light spots on the cervical tissue, also changes. Since this change of the shape of the two-dimensional pattern of light spots provides information about the orientation (both horizontally and vertically) of the colposcopy apparatus with respect to the cervix, it makes it easier to find a position in which the colposcopy apparatus is substantially perpendicular to the cervix, which may allow to image more of the cervix than would be visible under more oblique orientations of the colposcopy apparatus, where parts of the field of view (FOV) may be obstructed by a vaginal speculum.

It shall be noted that the provision of at least three light beam emitting units, as described above, can further be advantageous when the user wants to image the outermost regions of the cervix, that is, the regions that are present at the farthest end of the cervical channel. For instance, if the user wants to image the region at the 12 o' clock position of the cervix, keeping it in the center of the acquired image for a more detailed analysis of potential abnormalities, he/she would usually pan and tilt the colposcopy apparatus. The larger number of light beam emitting units can still allow using the positioning support provided by the emitted light beams even if one or more of the emitted light beams become obscured by the vaginal speculum when the colposcopy apparatus is panned and tilted by the user.

The optics unit can be an optics unit having a fixed focal distance corresponding to the predefined distance at which the emitted light beams intersect on the optical axis in front of the colposcopy apparatus. In this case, the imaging unit can be focused on the cervix by positioning the colposcopy apparatus such that the light spots produced by the emitted light beams on the cervical tissue are observed as a single light spot.

The image acquiring unit can comprise a CCD sensor with a suitable aspect ratio, pixel resolution and bit depth. For instance, a suitable CCD sensor can have a pixel resolution of 640 x 480 with an aspect ratio of 4:3 and a density of 96 dpi (dots per inch), a bit depth of 24 bit (8 bit per R, G, B color channel) and a video frame rate of 30 to 60 fps (frames per second). Of course, the image acquiring unit can also comprise another type of sensor, for instance, a suitable CMOS sensor (complementary metal oxide semiconductor) with comparable specifications.

The at least three light beam emitting units are preferably visual light beam emitting units, for instance, laser light beam emitting units, such as suitable laser diodes. However, also other types of light beam emitting units can be used as long as such units are able to emit suitably focused light beams. If non-coherent light is used, suitable focusing optics may be provided in order to focus the light into beams. It can be foreseen that the user can switch on and off the at least three light beam emitting units using a suitable user interface.

It is preferred to that the at least three light beam emitting units comprise four light beam emitting units that are arranged on the top, the left, the bottom, and the right with respect to the acquired image.

In particular, the four light beam emitting units can be arranged such that two of the emitted light beams lie in a first same plane with the optical axis and the other two of the emitted light beams lie in a second same plane with the optical axis, wherein the first same plane is arranged horizontally with respect to the acquired image and the second same plane is arranged vertically with respect to the acquired image. Such an arrangement of the four light beam emitting units is particularly suited for imaging the outermost regions of the cervix, as described above. Of course, the at least three light beam emitting units may also comprise more than four light beam emitting units, which can further improve the suitability of the colposcopy apparatus for imaging the outermost regions of the cervix.

It is further preferred that at least two of the emitted light beams have visually discernible colors.

This is particularly advantageous if the arrangement of the at least three light beam emitting units is pairwise symmetric with respect to the optical axis, that is, if respectively two of the emitted light beams lie in a same plane with the optical axis and are oriented under the same angle, since with such a symmetric arrangement the use of discernible colors for at least two of the emitted light beams provides information about whether the colposcopy apparatus is positioned too far away from or too close to the cervix.

For instance, let us assume a symmetric arrangement in which one of the emitted light beams has a color that is visually discernible from the color (or colors) of the other emitted light beams. The location of the light spot produced by that emitted light beam on the cervical tissue in the acquired image then depends on the distance of the colposcopy apparatus from the cervix. If the colposcopy apparatus is positioned too far away from the cervix, that is, the distance of the colposcopy apparatus from the cervix is larger than the predefined distance, the light spot is located in the acquired image at a first side relative to the location of the focal point (that is, the position at which the optical axis intersects the acquired image). On the other hand, if the colposcopy apparatus is positioned too close to the cervix, that is, the distance of the colposcopy apparatus from the cervix is smaller than the predefined distance, the light spot is located in the acquired image at a second side, opposite to the first side, relative to the position of the focal point. Thus, by determining on which side relative to the focal point the light spot is located, it can be determined whether the colposcopy apparatus is positioned too far away from or too close to the cervix.

The colors of the emitted light beams are preferably chosen such that they are easily visible to the human eye when seen against cervical tissue. In one preferred example, the at least three light beam emitting units are just three light beam emitting units and the emitted light beams have a red color, a green color, and a blue color, respectively. In this case, when the colposcopy apparatus is positioned at a distance from the cervix that equals the predefined distance, a single white light spot can be observed on the cervical tissue.

It is preferred that the at least three light beam emitting units are arranged on a circle having a predefined parameter and surrounding the optical axis, wherein the predefined distance is preferably between 23 cm and 27 cm with the predefined diameter being smaller than or equal to 4.6 cm to 5.4 cm, wherein the predefined distance is more preferably between 24 cm and 26 cm with the predefined diameter being smaller than or equal to 4.8 cm to 5.2 cm, wherein the predefined distance is most preferred 25 cm with the predefined diameter being smaller than or equal to 5 cm.

A predefined distance of 25 cm is particularly advantageous since it allows the colposcopy apparatus to be far enough away from the cervix such that other clinical instruments like a cotton swab, stains, therapy devices and the like can easily be used when performing the colposcopy procedure, but still close enough to the cervix such that the user can comfortably perform the colposcopy procedure, even if he/she has comparably short arms. With this predefined distance, the predefined diameter is preferably smaller than or equal to 5 cm since in this case all the emitted light beams can converge on the cervix without becoming obstructed by a vaginal speculum having a typical inner diameter of 1.6 cm and a length of 8 cm (corresponding to a normal depth of the vaginal channel). Of course, the predefined diameter should suitably be larger than 0 cm, for instance, larger than 1 cm or larger than 3 cm.

The upper values for the predefined diameter have been calculated above according to the following equation $D^{laser} = d \cdot (D^{speculum} / D^{vaginal\_channel})$, where $D^{laser}$ represents the predefined diameter, d represents the predefined distance, $D^{speculum}$ represents the inner diameter of the vaginal speculum and $D^{vaginal\_channel}$, represents the depth of the vaginal channel. With this equation, other combinations of the predefined distance and the predefined parameter may be calculated, for instance, for vaginal specula having different (smaller or larger) inner diameters.

The colposcopy apparatus may further comprise:

- an image analysis unit for analysing the acquired image of the cervix with respect to light spots produced by the emitted light beams on the cervical tissue to determine information about the positioning of the colposcopy apparatus with respect to the cervix,
- a user indication unit for providing indications to a user of the colposcopy apparatus to support the user in positioning the colposcopy apparatus based on the determined information.

By providing such an image analysis unit, the information provided by the two-dimensional pattern of light spots produced by the emitted light beams on the cervical tissue can be analysed and, preferably, quantified in the acquired image. It can then be possible when the colposcopy apparatus is aimed at the cervical tissue during the process of positioning the colposcopy apparatus to - qualitatively or quantitatively - determine the distance of the colposcopy apparatus from the cervix and/or the orientation of the colposcopy apparatus with respect to the cervix. Based on the determined information about the positioning of the colposcopy apparatus with respect to the cervix, the user of the colposcopy apparatus can then be provided with indications supporting him/her in positioning the colposcopy apparatus.

The user indication unit can be adapted for displaying the acquired image to the user and to visually provide the indications with or in addition to the displayed image, for instance, as a visual overlay provided on the displayed image. However, it is also possible that the user indication unit is adapted to provide the indications to the user by other means, for example, in an audible or tactile manner. In these cases, the user indication unit can comprise suitable indication elements, such as one or more audio transducer elements or a vibration element.

It is preferred that the indications comprise first indications indicating whether the colposcopy apparatus must be moved towards the cervix or away from the cervix to position the colposcopy apparatus at a distance from the cervix equal to the predefined distance.

Such first indications about the direction for moving the colposcopy apparatus are particularly useful for supporting the user in quickly positioning the colposcopy apparatus at a distance from the cervix equal to the predefined distance. The first indications can be plain directional indications like "forwards" and "backwards", but they can also comprise or consist of quantitative information, for instance, they can give the deviation from the predefined distance in terms of positive and negative numerical values, such as -2.5 cm or +0.7 cm. Of course, it is also possible that the first indications make use of signs that are associated with certain movement directions. For instance, a green light may be associated with a situation in which the colposcopy apparatus must be moved towards the cervix to position the colposcopy apparatus at a distance from the cervix equal to the predefined distance and a red light may be associated with a situation in which the colposcopy apparatus must be moved away from the cervix.

It is further preferred that the indications comprise second indications indicating whether the colposcopy apparatus is positioned such that the cervix is within the depth of field (DOF) of the imaging unit.

The DOF of the imaging unit is the range of distances in front of the colposcopy apparatus for which, if the colposcopy apparatus is positioned within this range of distances from the cervix, the acquired image is still acceptably well focused. The size of the DOF depends on the specifics of the imaging unit, in particular, on the characteristics of the optics unit and the image acquiring unit. Such second indications about the positioning of the colposcopy apparatus are particularly useful since they allow the user to tolerate a certain deviation when trying to position the colposcopy apparatus at a distance from the cervix equal to the predefined distance and still acquire a sharp image. The second indications can be plain indications like "in" and "out" (of the DOF), but they can also make use of signs that are associated with the cervix being either within or out-of the DOF. Preferably, however, the user indication unit is adapted to display the acquired image to the user, wherein the second indications comprise a visual representation of the DOF displayed with the acquired image. For instance, in the case that the at least three light beam emitting units are arranged on a circle having a predefined parameter and surrounding the optical axis, the DOF can be visually represented by a circle overlaid on the displayed image. Therewith, it can easily be decided whether the cervix is within or out-of the DOF. If the light spots produced by the emitted light beams are within the circle, the cervix is within the DOF; if they are out-of the circle, the cervix is out-of the DOF.

The colposcopy apparatus may further comprise:

-    an image analysis unit for analysing the acquired image of the cervix with respect to light spots produced by the emitted light beams on the cervical tissue to determine the distance of the colposcopy apparatus from the cervix,

wherein the colposcopy apparatus is adapted to adjust the focal distance and/or the zoom of the imaging unit based on the determined distance.

By providing such an image analysis unit, the information provided by the two-dimensional pattern of light spots produced by the emitted light beams on the cervical tissue can be analysed in the acquired image and the distance of the colposcopy apparatus from the cervix can be determined. By adjusting the focal distance and/or the zoom of the imaging unit based on the determined distance, it can then be possible to acquire a sharp image of the cervix, preferably showing the cervix such that it substantially just completely fills the acquired image, even if the colposcopy apparatus is positioned at a distance from the cervix different from the predefined distance. This can be particularly helpful, for instance, when the user pans and tilts the colposcopy apparatus to image the outermost regions of the cervix, as described above, since due to the panning and tilting the positioning of the colposcopy apparatus may deviate from the predefined distance.

The adjustment of the focal distance and/or the zoom of the imaging unit may comprise purely optical techniques or a combination of optical and digital techniques. For instance, the optics unit may comprise at least one variable-focus lens based, for instance, on liquids, for adjusting the focal distance of the imaging unit and the adjustment of the zoom may be performed by means of digital zooming and/or by the use of different zoom lenses. The colposcopy apparatus may comprise a predefined look-up table storing for a range of determined distances of the colposcopy apparatus from the cervix suitable values for adjusting the focal distance and/or the zoom of the imaging unit. The range of determined distances may comprise, for instance, distances of the colposcopy apparatus from the cervix ranging from 20 cm to 30 cm.

It can also be possible that different sets of values for adjusting the zoom of the imaging unit are provided within the colposcopy apparatus for choosing between different magnifications of the cervix. For instance, with a first set of values the cervix may always be shown such that it substantially just completely fills the acquired image whereas with a second set of values the cervix may always be shown with twice the magnification, effectively resulting in only a magnified fraction of the cervix being shown in the acquired image. Of course, it can be foreseen that the user can select between such different sets of values for adjusting the zoom of the imaging unit using a suitable user interface. Also, it shall be understood that with different sets of values for adjusting the zoom also corresponding different sets of values for adjusting the focal distance may be provided.

It is preferred that for changes in the determined distance, the focal distance and/or the zoom of the imaging unit are adjusted such that the size of the cervix or of a fraction thereof shown in the acquired image is kept constant.

This has the advantage that the user can be constantly provided with a sharp acquired image showing the cervix or a fraction thereof at the same size even if changes in the distance between the colposcopy apparatus and the cervix occur during the colposcopy procedure, for instance, due to slight movements of the patient. It can therefore be easier for the user to visually check the surface of the cervix for abnormalities.

There is also described herein a colposcopy apparatus for performing a colposcopy procedure, wherein the colposcopy apparatus comprises:

- an imaging unit comprising an optics unit having an optical axis and an image acquiring unit for acquiring an image of a cervix through the optics unit,
- an infrared light distance sensing unit comprising an infrared light beam emitter and an infrared light receiver spaced apart from the optical axis, wherein the infrared light beam emitter is adapted to emit an infrared light beam that is coplanar with the optical axis and that intersects on the optical axis at a predefined distance in front of the colposcopy apparatus, wherein the infrared light receiver lies in a same plane with the emitted infrared light beam and the optical axis and is adapted to receive the emitted infrared light beam after it has been reflected, and wherein the infrared light distance sensing unit is adapted to sense a distance based on the emitted and reflected infrared light beam, and
- a user indication unit for providing indications to a user of the colposcopy apparatus to support the user in positioning the colposcopy apparatus based on the currently sensed distance.

It has occurred to the inventors that experienced users appear to be generally quite good in roughly positioning the colposcopy apparatus at a proper working distance, for instance, 25 cm (cf. above), from the cervix, but that it is more difficult to position the colposcopy apparatus such that it is actually aimed at the cervix. In order to support the user in this later respect, prior knowledge about the patient's anatomy of interest can be used. In particular, it is the case that the cervix is located at the farthest end of the vaginal canal (which normally has a depth of about 8 cm). Thus, if the user roughly positions the colposcopy apparatus at the proper working distance, the currently sensed distance should be substantially equal to the proper working distance if the colposcopy apparatus is aimed at the cervix, whereas it should generally be much smaller if the colposcopy apparatus is not aimed at the cervix, for instance, if it is aimed at a vaginal speculum, at another body part of the patient, et cetera. This effect can be used by the user indication unit for providing indications to the user to support the user in positioning the colposcopy apparatus.

The indications can comprise or consist of quantitative information, for instance, they can give the currently sensed distance in terms of a numerical value, such as 23.7 cm or 18.2 cm. Given this information, the user can then be able to tell based on his/her experience whether the colposcopy apparatus is aimed at the cervix or not. Alternatively, the indications can also be plain indications like "Yes" and "No", wherein "Yes" means that the colposcopy apparatus is aimed at the cervix and "No" means that the colposcopy apparatus is not aimed at the cervix. These two situations may be distinguished by the user indication unit by comparing the currently sensed distance with a suitably defined threshold, for instance, 20 cm. Of course, instead of plain indications, one can also make use of signs, such as a green light and a red light, that are associated with these two situations.

The user indication unit can be adapted for displaying the acquired image to the user and to visually provide the indications with or in addition to the displayed image, for instance, as a visual overlay provided on the displayed image. However, it is also possible that the user indication unit is adapted to provide the indications to the user by other means, for example, in an audible or tactile manner. In these cases, the user indication unit can comprise suitable indication elements, such as one or more audio transducer elements or a vibration element.

The image acquiring unit can comprise a CCD sensor with a suitable aspect ratio, pixel resolution and bit depth. For instance, a suitable CCD sensor can have a pixel resolution of 640 x 480 with an aspect ratio of 4:3 and a density of 96 dpi (dots per inch), a bit depth of 24 bit (8 bit per R, G, B color channel) and a video frame rate of 30 to 60 fps (frames per second). Of course, the image acquiring unit can also comprise another type of sensor, for instance, a suitable CMOS sensor (complementary metal oxide semiconductor) with comparable specifications.

The colposcopy apparatus may be adapted to provide an initialization phase during which the infrared light distance sensing unit repeatedly senses the distance to generate a plurality of sensed distances, while a user of the colposcopy apparatus shall move the colposcopy apparatus in substantially a plane in front of the cervix, to determine a working distance of the colposcopy apparatus from the cervix based on the plurality of sensed distances, and to store the working distance, wherein the indications are further based on the stored working distance.

The infrared light distance sensing unit senses the distance based on the reflected infrared light beam. However, there is generally no direct indication at which surface - of the cervix, of the vaginal speculum, of another body part of the patient, et cetera - the infrared light beam has been reflected, that is, to which object the sensed distance actually relates. In order to make the positioning support more robust, in particular, for less experiences users who are not that good in positioning the colposcopy apparatus at a proper working distance from the cervix, for instance, who are not able to tell with good accuracy how far away from the cervix they have positioned the colposcopy apparatus, the initialization phase can be performed on the patient before performing the actual colposcopy procedure. In this phase, when the user moves the colposcopy apparatus in substantially a plane in front of the cervix, the "actual" working distance of the colposcopy apparatus from the cervix can be determined based on the plurality of sensed distances. In particular, due to the above-discussed characteristics of the patient's anatomy of interest, the working distance of the colposcopy apparatus will ideally correspond to the largest sensed distance from among the plurality of sensed distances. This distance may therefore be stored as the working distance of the colposcopy apparatus from the cervix. The indications provided by the user indication unit to the user to support the user in positioning the colposcopy apparatus can then be further based on the stored working distance. For instance, they can comprise or consist of quantitative information, for instance, they can give the deviation of the currently sensed distance from the stored working distance in terms of positive and negative numerical values, such as -1.5 cm or +0.9 cm. Given this information, the user can then be able to more easily tell whether the colposcopy apparatus is aimed at the cervix or not.

It is preferred that the colposcopy apparatus further comprises:

- an image analysis unit for analysing the acquired image of the cervix to determine information about the focal distance and/or the zoom of the imaging unit,

wherein the colposcopy apparatus is adapted, during the initialization phase, when the colposcopy apparatus is positioned with respect to the cervix such that the currently sensed distance matches the stored working distance, to adjust the focal distance and/or the zoom of the imaging unit based on the determined information for the currently acquired image of the cervix and, after adjusting the focal distance and/or the zoom of the imaging unit, to store the currently acquired image of the cervix.

The information about the focal distance and/or the zoom of the imaging unit can be determined, for instance, by image analysis techniques that are able to assess an amount of blur of the currently acquired image and/or to detect the cervix or a fraction thereof in the currently acquired image. Based on the assessed amount of blur of the currently acquired image, the focal distance of the imaging unit may then be iteratively adjusted. Additionally or alternatively, the zoom of the imaging unit may be adjusted, for instance, iteratively, based on the detected cervix or fraction thereof in the currently acquired image. By adjusting the focal distance and/or the zoom of the imaging unit, it can possible to acquire a sharp image of the cervix, preferably showing the cervix such that it substantially just completely fills the acquired image, and to store this image as a kind of "gold standard" for later acquired images of the cervix.

The adjustment of the focal distance and/or the zoom of the imaging unit may comprise purely optical techniques or a combination of optical and digital techniques. For instance, the optics unit may comprise at least one variable-focus lens based, for instance, on liquids, for adjusting the focal distance of the imaging unit and the adjustment of the zoom may be performed by means of digital zooming and/or by the use of different zoom lenses.

It is further preferred that the image analysis unit is adapted to determine further information about a difference between the currently acquired image of the cervix and the stored image of the cervix, and wherein the colposcopy apparatus is adapted to provide a colposcopy procedure phase during which the focal distance and/or the zoom of the imaging unit are adjusted based on the determined further information.

By adjusting, during the "actual" colposcopy procedure phase, the focal distance and/or the zoom of the imaging unit based on the determined further information, it can be possible to acquire a sharp image of the cervix, preferably showing the cervix such that it substantially just completely fills the acquired image, even if the colposcopy apparatus is positioned at a distance from the cervix different from the stored working distance. This can be particularly helpful, for instance, since due to the different steps performed as part of the colposcopy procedure (that is, cleaning, Aceto-whiteness test, iodine uptake test, et cetera), the positioning of the colposcopy apparatus may deviate from the stored working distance. The further information about a difference between the currently acquired image of the cervix and the stored image of the cervix can be determined, for instance, by image analysis techniques that perform a matching or correlation between the currently acquired image of the cervix and the stored image of the cervix, wherein the focal distance and/or the zoom of the imaging unit can be adjusted to minimize the difference. Since, during the colposcopy procedure phase, the currently acquired image due of the cervix also varies in color due to the application of the diluted acetic acid and the 5 % Lugol's iodine, the matching or the correlation should be robust to such variations. For instance, the matching or the correlation may be performed in a transformed domain (cf., for instance, Matungka R. et al., "Image Registration Using Adaptive Polar Transform", in IEEE Transactions on Inage Processing, Vol. 18, No. 10, October 2009, pages 2340 to 2354).

The colposcopy apparatus may be adapted to adjust the focal distance and/or the zoom of the imaging unit based on the currently sensed distance.

Also by adjusting the focal distance and/or the zoom of the imaging unit based on the currently sensed distance, it can be possible to acquire a sharp image of the cervix, preferably showing the cervix such that it substantially just completely fills the acquired image. The colposcopy apparatus may comprise a predefined look-up table storing for a range of currently sensed distances of the colposcopy apparatus from the cervix suitable values for adjusting the focal distance and/or the zoom of the imaging unit. The range of currently sensed distances may comprise, for instance, distances of the colposcopy apparatus from the cervix ranging from 20 cm to 30 cm.

It can also be possible that different sets of values for adjusting the zoom of the imaging unit are provided within the colposcopy apparatus for choosing between different magnifications of the cervix. For instance, with a first set of values the cervix may always be shown such that it substantially just completely fills the acquired image whereas with a second set of values the cervix may always be shown with twice the magnification, effectively resulting in only a magnified fraction of the cervix being shown in the acquired image. Of course, it can be foreseen that the user can select between such different sets of values for adjusting the zoom of the imaging unit using a suitable user interface. Also, it shall be understood that with different sets of values for adjusting the zoom also corresponding different sets of values for adjusting the focal distance may be provided.

It is preferred that the infrared light beam emitter and the infrared light receiver are arranged on a circle having a predefined diameter and surrounding the optical axis, wherein the predefined distance is preferably between 23 cm and 27 cm with the predefined diameter being smaller than or equal to 4.6 cm to 5.4 cm, wherein the predefined distance is more preferably between 24 cm and 26 cm with the predefined diameter being smaller than or equal to 4.8 cm to 5.2 cm, wherein the predefined distance is most preferred 25 cm with the predefined diameter being smaller than or equal to 5 cm.

A predefined distance of 25 cm is particularly advantageous since it allows the colposcopy apparatus to be far enough away from the cervix such that other clinical instruments like a cotton swab, stains, therapy devices and the like can easily be used when performing the colposcopy procedure, but still close enough to the cervix such that the user can comfortably perform the colposcopy procedure, even if he/she has comparably short arms. With this predefined distance, the predefined diameter is preferably smaller than or equal to 5 cm since in this case the emitted infrared light beam can converge on the cervix without becoming obstructed by a vaginal speculum having a typical inner diameter of 1.6 cm and a length of 8 cm (corresponding to a normal depth of the vaginal channel). Of course, the predefined diameter should suitably be larger than 0 cm, for instance, larger than 1 cm or larger than 3 cm.

The upper values for the predefined diameter have been calculated above according to the following equation $D^{infrared} = d \cdot (D^{speculum} / D^{vaginal\text{-}channel})$, where $D^{infrared}$ represents the predefined diameter, d represents the predefined distance, $D^{speculum}$ represents the inner diameter of the vaginal speculum and $D^{vaginal\_channel}$ represents the depth of the vaginal channel. With this equation, other combinations of the predefined distance and the predefined parameter may be calculated, for instance, for vaginal specula having different (smaller or larger) inner diameters.

It shall be understood that the colposcopy apparatus for performing a colposcopy procedure of claim 1 and the colposcopy apparatus for performing a colposcopy procedure of claim 11 have similar and/or identical preferred embodiments as defined in the dependent claims.

It shall be understood that a preferred embodiment of the invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily a first embodiment of a colposcopy apparatus for performing a colposcopy procedure,
Fig. 2 shows schematically and exemplarily a situation in which the outermost regions of the cervix are imaged with the colposcopy apparatus shown in Fig. 1,
Fig. 3 shows schematically and exemplarily shows how second indications are used to indicate whether the colposcopy apparatus shown in Fig. 1 is positioned such that the cervix is within the DOF of the imaging unit,
Fig. 4 shows a graph exemplarily illustrating suitable values for adjusting the focal distance and/or the zoom of the imaging unit of the colposcopy apparatus shown in Fig. 1 based on the determined distance of the colposcopy apparatus from the cervix, and

Fig. 5 shows schematically and exemplarily a second embodiment of a colposcopy apparatus for performing a colposcopy procedure.

DETAILED DESCRIPTION OF EMBODIMENTS

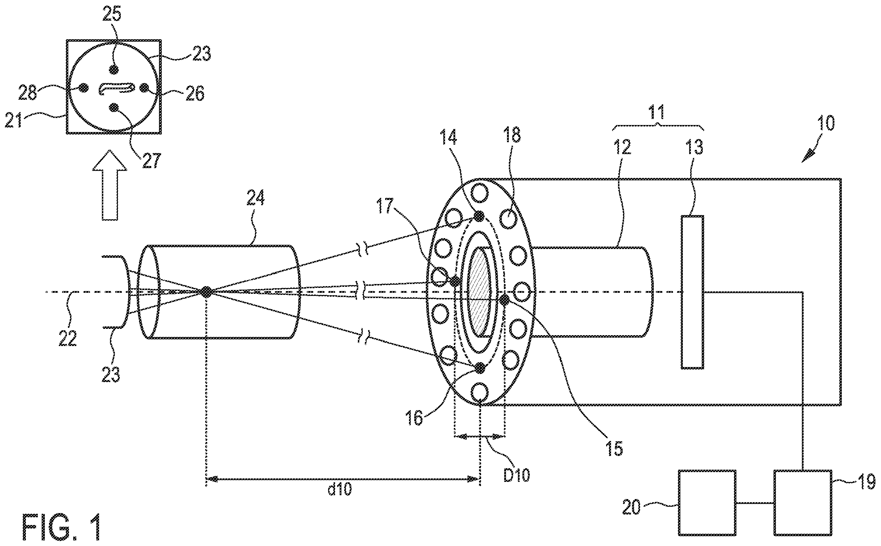

Fig. 1 shows schematically and exemplarily a first embodiment of a colposcopy apparatus 10 for performing a colposcopy procedure. The colposcopy apparatus 10 comprises an imaging unit 11 which comprises an optics unit 12 having an optical axis 22 and an image acquiring unit 13 for acquiring an image 21 of a cervix 23 through the optics unit 12. The colposcopy apparatus 10 further comprises at least three, here, four light beam emitting units 14, 15, 16, 17 which are spaced apart from the optical axis 22. The four light beam emitting units 14, 15, 16, 17 are adapted to emit light beams that are coplanar with the optical axis 22 and that intersect on the optical axis 22 at a predefined distance

d10 in front of the colposcopy apparatus 10, wherein not all of the emitted light beams lie in a same plane. In this example, the four light beam emitting units 14, 15, 16, 17 are arranged at the front end of the colposcopy apparatus 10, that is, the end that is aimed at the cervix 23 when performing the colposcopy procedure, here, at a ring-shaped element that also contains a plurality of lighting elements 18 for lighting the cervix 23.

When the colposcopy apparatus 10 is aimed at cervical tissue during the process of positioning the colposcopy apparatus 10, the emitted light beams produce a two-dimensional pattern of light spots 25, 26, 27, 28 on the tissue. The overall dimension of this two-dimensional pattern of light spots 25, 26, 27, 28 becomes smaller - ultimately converging into a single observable light spot - when the distance of the colposcopy apparatus 10 from the cervix 23 approaches the predefined distance d10, that is, the distance at which the emitted light beams intersect on the optical axis 22 in front of the colposcopy apparatus 10, making it easy to position the colposcopy apparatus 10 at the predefined distance d10 from the cervix 23. In addition, when the orientation of the colposcopy apparatus 10 changes with respect to the cervix 23, the shape of the two-dimensional pattern of light spots 25, 26, 27, 28, that is, the relative distances between the light spots 25, 26, 27, 28 on the cervical tissue, also changes. Since this change of the shape of the two-dimensional pattern of light spots 25, 26, 27, 28 provides information about the orientation (both horizontally and vertically) of the colposcopy apparatus 10 with respect to the cervix 23, it makes it easier to find a position in which the colposcopy apparatus 10 is substantially perpendicular to the cervix 23, which may allow to image more of the cervix 23 than would be visible under more oblique orientations of the colposcopy apparatus 10, where parts of the FOV may be obstructed by a vaginal speculum 24.

Here, the optics unit 12 has a fixed focal distance corresponding to the predefined distance d10 at which the emitted light beams intersect on the optical axis 22 in front of the colposcopy apparatus 10. The imaging unit 11 can thus be focused on the cervix 23 by positioning the colposcopy apparatus 10 such that the light spots 25, 26, 27, 28 produced by the emitted light beams on the cervical tissue are observed as a single light spot.

The image acquiring unit 13, here, comprises a CCD sensor having a pixel resolution of 640 x 480 with an aspect ratio of 4:3 and a density of 96 dpi, a bit depth of 24 bit (8 bit per R, G, B color channel) and a video frame rate of 30 to 60 fps.

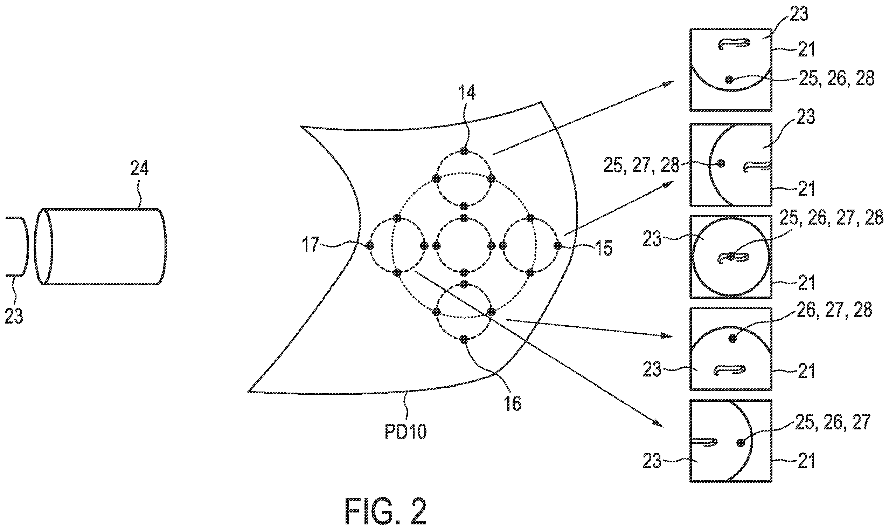

In this embodiment, the four light beam emitting units 14, 15, 16, 17 arranged on the top, the left, the bottom, and the right with respect to the acquired image 21. In particular, the four light beam emitting units 14, 15, 16, 17 are arranged such that two of the emitted light beams lie in a first same plane (not shown in the figure) with the optical axis 22 and the other two of the emitted light beams lie in a second same plane (not shown in the figure) with the optical axis 22, wherein the first same plane is arranged horizontally with respect to the acquired image 21 and the second same plane is arranged vertically with respect to the acquired image 21. As will be described next in more detail with reference to Fig. 2, such an arrangement of the four light beam emitting units 14, 15, 16, 17 is particularly suited for imaging the outermost regions of the cervix 23, that is, the regions that are present at the farthest end of the cervical channel. The four light beam emitting units 14, 15, 16, 17, here, are arranged on a circle (shown in the figure as a stippled line) having a predefined diameter D10 and surrounding the optical axis 22.

Fig. 2 shows schematically and exemplarily a situation in which the outermost regions of the cervix 23 are imaged with the colposcopy apparatus 10 shown in Fig. 1. Here, in order to image the regions at the 12, 3, 6 and 9 o' clock positions of the cervix 23, keeping them in the center of the acquired image 21 for a more detailed analysis of potential abnormalities, the user pans and tilts the colposcopy apparatus 10. If, in doing so, he/she also takes care to position the colposcopy apparatus 10 at the predefined distance d10 from the cervix 23 in order to focus the imaging unit 11 on the cervix 23, the positioning of the colposcopy apparatus 10 defines a slightly curved "predefined distance plane" PD10. As can further be seen from the figure, when imaging the regions at the 12, 3, 6 and 9 o' clock positions of the cervix 23 as described above, in each case one or more, here, one of the emitted light beams (not shown in the figure) may become obscured by the vaginal speculum 24 when the colposcopy apparatus 10 is panned and tilted by the user. This is visualized by the large dotted circle drawn in the predefined distance plane. In more detail, in this example, when imaging the 6 o' clock position of the cervix 23, the light beam emitted by the light beam emitting unit 14 becomes obscured by the vaginal speculum 24. In contrast, the light beams emitted from the three other light beam emitting units 15, 16, 17 do not become obscured by the vaginal speculum 24 and, thus, can still allow using the positioning support. Indeed, as can be understood from the corresponding acquired image 21, which is illustrated on the top right of the figure, the non-obscured light beams emitted by the light beam emitting units 15, 16, 17 still produce a two-dimensional pattern of light spots 25, 26, 28 on the cervical tissue (which converges into a single observable light spot when the imaging unit 11 is properly focused on the cervix 23). The same considerations apply when imaging the 9 o' clock position of the cervix 23, in which case the light beam emitted by the light beam emitting unit 15 becomes obscured by the vaginal speculum 24, when imaging the 12 o' clock position of the cervix 23, in which case the light beam emitted by the light beam emitting unit 16 becomes obscured by the vaginal speculum 24, and when imaging the 3 o' clock position of the cervix 23, in which case the light beam emitted by the light beam emitting unit 17 becomes obscured by the vaginal speculum 24. In each case, however, the other three light beam emitting units still allow using the positioning support, as can be understood from the corresponding acquired images 21.

With returning reference to Fig. 1, in this embodiment, the predefined distance d10 is 25 cm with the predefined diameter D10 being smaller than or equal to 5 cm. With this combination of values for the predefined distance d10 and the predefined diameter D10 all the emitted light beams can converge on the cervix 23 without becoming obstructed by the vaginal speculum 24 having a typical inner diameter of 1.6 cm and a length of 8 cm (corresponding to a normal depth of the vaginal channel). Of course, the predefined diameter D10 should suitably be larger than 0 cm, for instance, larger than 1 cm or larger than 3 cm. For instance, here, the predefined diameter D10 is 4 cm.

The arrangement of the four light beam emitting units 14, 15, 16, 17 is pairwise symmetric with respect to the optical axis 22, that is, respectively two of the emitted light beams lie in a same plane with the optical axis 22 and are oriented under the same angle. In this embodiment, at least two, here, all of the emitted light beams have visually discernible colors, since with such a symmetric arrangement the use of discernible colors for at least two of the emitted light beams provides information about whether the colposcopy apparatus 10 is positioned too far away from or too close to the cervix 23. The colors of the emitted light beams are chosen such that they are easily visible to the human eye when seen against cervical tissue.

In this embodiment, the colposcopy apparatus 10 further comprises an image analysis unit 19 for analysing the acquired image 21 of the cervix 23 with respect to light spots 25, 26, 27, 28 produced by the emitted light beams on the cervical tissue to determine information about the positioning of the colposcopy apparatus 10 with respect to the cervix 23, and a user indication unit 20 for providing indications to a user of the colposcopy apparatus 10 to support the user in positioning the colposcopy apparatus 10 based on the determined information. The user indication unit 20, here, is adapted for displaying the acquired image 21 to the user and to visually provide the indications as a visual overlay (not shown in the figure) provided on the displayed image.

The indications comprise first indications indicating whether the colposcopy apparatus 10 must be moved towards the cervix 23 or away from the cervix 23 to position the colposcopy apparatus 10 at a distance from the cervix 23 equal to the predefined distance d10. In this example, the first indications consist of quantitative information giving the deviation from the predefined distance d10 in terms of positive and negative numerical values, such as -2.5 cm or +0.7 cm. In other examples, the first indications can also be plain directional indications like "forwards" and "backwards" or they can make use of signs that are associated with certain movement directions.

In this embodiment, the indications further comprise second indications indicating whether the colposcopy apparatus 10 is positioned such that the cervix 23 is within the DOF of the imaging unit 11. In this example, the second indications comprise a visual representation, here, a circle, of the DOF (not shown in the figure) overlaid on the displayed image. In other examples, the second indications can be plain indications like "in" and "out" (of the DOF) or they can make use of signs that are associated with the cervix being either within or out-of the DOF.

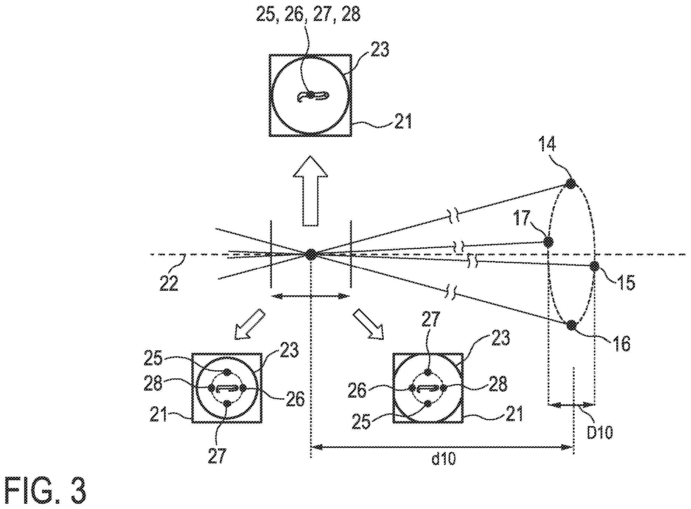

This will be described in more detail with reference to Fig. 3 which shows schematically and exemplarily how second indications are used to indicate whether the colposcopy apparatus 10 shown in Fig. 1 is positioned such that the cervix 23 is within the DOF of the imaging unit 11. The figure is similar to Fig. 1 but shows only the four light beam emitting units 14, 15, 16, 17 as well as the light beams emitted therefrom. The DOF of the imaging unit 11 is the range of distances in front of the colposcopy apparatus 10 for which, if the colposcopy apparatus 10 is positioned within this range of distances from the cervix 23, the acquired image 21 is still acceptably well focused. The size of the DOF depends on the specifics of the imaging unit 11, in particular, on the characteristics of the optics unit 12 and the image acquiring unit 13 (all not shown in the figure). Here, the DOF is symmetrical with respect to the predefined distance d10. As can be understood from Fig. 3, if the colposcopy apparatus 10 is positioned at the predefined distance d10 from the cervix 23, the light spots 25, 26, 27, 28 produced by the emitted light beams on the cervical tissue are observed as a single light spot. In contrast, if the colposcopy apparatus 10 is positioned at a distance different from the predefined distance d10 from the cervix 23, the light spots 25, 26, 27, 28 produced by the emitted light beams produce a two-dimensional pattern of light spots 25, 26, 27, 28 on the tissue. If the DOF is visually represented by a circle overlaid on the displayed image 21 (in the figure shown as a stippled circle), it can easily be decided whether the cervix 23 is within or out-of the DOF. If the light spots 25, 26, 27, 28 produced by the emitted light beams are within the circle, the cervix 23 is within the DOF; if they are out-of the circle, the cervix 23 is out-of the DOF.

While in the first embodiment described with reference to Fig. 1 above, the optics unit 12 has a fixed focal distance corresponding to the predefined distance d10 at which the emitted light beams intersect on the optical axis 22 in front of the colposcopy apparatus 10, in other embodiments, the optics unit 12 may comprise at least one variable-focus lens based, for instance, on liquids, for adjusting the focal distance of the imaging unit 11. In this case, the image analysis unit 19 may - additionally or alternatively - be adapted to analyse the acquired image 21 of the cervix 23 with respect to light spots 25, 26, 27, 28 produced by the emitted light beams on the cervical tissue to determine the distance of the colposcopy apparatus 10 from the cervix 23, wherein the colposcopy apparatus 10 may be adapted to adjust the focal distance and/or the zoom of the imaging unit 11 based on the determined distance.

By adjusting the focal distance and/or the zoom of the imaging unit 11 based on the determined distance, it can then possible to acquire a sharp image of the cervix 23, preferably showing the cervix 23 such that it substantially just completely fills the acquired image 21, even if the colposcopy apparatus 10 is positioned at a distance from the cervix 23 different from the predefined distance d10. This can be particularly helpful, for instance, when the user pans and tilts the colposcopy apparatus 10 to image the outermost regions of the cervix 23, as described above, since due to the panning and tilting the positioning of the colposcopy apparatus 10 may deviate from the predefined distance d10.

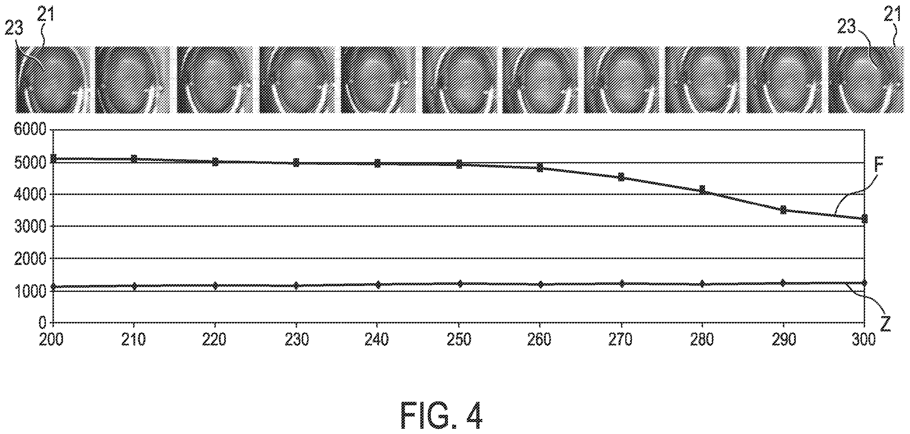

This will be described in more detail with reference to Fig. 4 which shows a graph exemplarily illustrating suitable values for adjusting the focal distance and/or the zoom of the imaging unit 11 of the colposcopy apparatus 10 shown in Fig. 1 based on the determined distance of the colposcopy apparatus 10 from the cervix 23. As can be seen from Fig. 4, for a range of determined distances, here, distances of the colposcopy apparatus 10 from the cervix 23 ranging from 20 cm to 30 cm (x-axis), suitable values for adjusting the focal distance and the zoom of the imaging unit 11 have been calculated (y-axis). In this example, the values are specific to the Goldway SLC-2000B digital video colposcope. They comprise a "focus value" F for adjusting the focal distance and a "zoom value" Z for adjusting the zoom of the imaging unit 11. These values have been calculated according to the following equations:

$$\text{Focus value: } F = \text{sqrt}(-3.1209d^2 + 1382.7d - 101703), \qquad R^2 = 0.9729$$

$$\text{Zoom value: } Z = 12.5d + 8650, \qquad\qquad R^2 = 1$$

In these equations, d represents the distance of the colposcopy apparatus 10 from the cervix 23, F represents the corresponding focus value, sqrt() denotes the square root operation, Z represents the corresponding zoom value and $R^2$ is a quantifying parameter to showcase a "goodness of fit". (The value ranges from 0, which is the worst fit, to 1, which is best fit. This means that the closer the value is to 1, the better the curve fits to the data points.)

The values for adjusting the focal distance and the zoom of the imaging unit 11 have been calculated, in this example, such that the size of the cervix 23 shown in the acquired image 21 is kept constant (here, particularly, such that the cervix 23 substantially just completely fills the acquired image 21) for changes in the determined distance, as shown at the top of the figure. This has the advantage that the user can be constantly provided with a sharp acquired image showing the cervix 23 or a fraction thereof at the same size even if changes in the distance between the colposcopy apparatus 10 and the cervix 23 occur during the colposcopy procedure, for instance, due to slight movements of the patient. It can therefore be easier for the user to visually check the surface of the cervix 23 for abnormalities.

The calculated values for adjusting the focal distance and the zoom of the imaging unit 11 are also shown in the following Table 1.

Table 1 - Calculated values for adjusting the focal distance and the zoom

| Dist. | 200 | 210 | 220 | 230 | 240 | 250 | 260 | 270 | 280 | 290 | 300 |
|---|---|---|---|---|---|---|---|---|---|---|---|
| F | 51248 | 51098 | 50100 | 50020 | 49620 | 49320 | 48320 | 45370 | 41260 | 35260 | 32248 |
| Z | 11150 | 11275 | 11400 | 11525 | 11650 | 11775 | 11900 | 12025 | 12150 | 12275 | 12400 |

With returning reference to Fig. 1, the adjustment of the focal distance and the zoom of the imaging unit 11 may comprise purely optical techniques or a combination of optical and digital techniques. For instance, as mentioned above, the optics unit 12 may comprise at least one variable-focus lens based, for instance, on liquids, for adjusting the focal distance of the imaging unit 11 and the adjustment of the zoom may be performed by means of digital zooming and/or by the use of different zoom lenses.

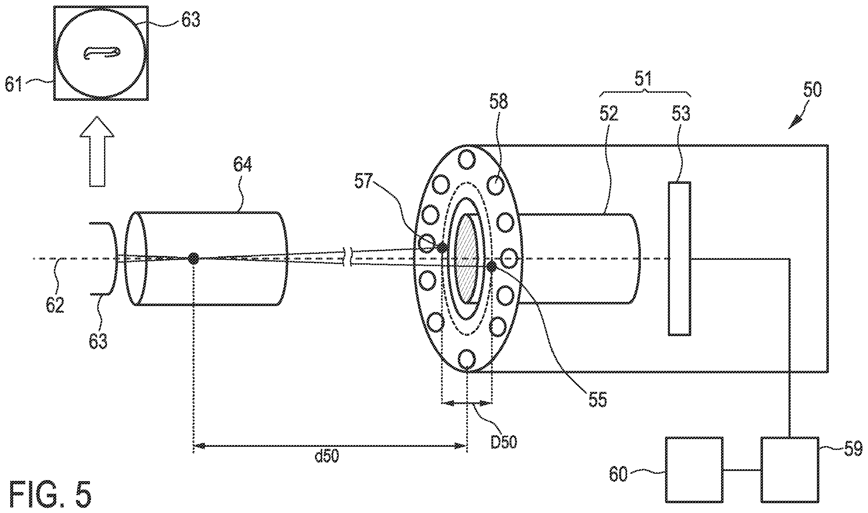

Fig. 5 shows schematically and exemplarily a second embodiment of a colposcopy apparatus 50 for performing a colposcopy procedure. The colposcopy apparatus 50 is similar to the colposcopy apparatus 10 shown in Fig. 1. In particular, it also comprises an imaging unit 51 which comprises an optics unit 52 having an optical axis 62 and an image acquiring unit 53 for acquiring an image 61 of a cervix 63 through the optics unit 52. In contrast to the colposcopy apparatus 10, however, the colposcopy apparatus 50 comprises an infrared light distance sensing unit 55, 57 comprising an infrared light beam emitter 55 and an infrared light receiver 57 spaced apart from the optical axis 62. The infrared light beam emitter 55 is adapted to emit an infrared light beam that is coplanar with the optical axis 62 and that intersects on the optical axis 62 at a predefined distance d50 in front of the colposcopy apparatus 50, and the infrared light receiver 57 lies in a same plane with the emitted infrared light beam and the optical axis 62 and is adapted to receive the emitted infrared light beam after it has been reflected. The infrared light distance sensing unit 55, 57 is adapted to sense a distance based on the emitted and reflected infrared light beam. In this example, the infrared light beam emitter 55 and the infrared light receiver 57 are arranged at the front end of the colposcopy apparatus 50, that is, the end that is aimed at the cervix 63 when performing the colposcopy procedure, here, at a ring-shaped element that also contains a plurality of lighting elements 58 for lighting the cervix 63.

The image acquiring unit 53, here, comprises a CCD sensor having a pixel resolution of 640 x 480 with an aspect ratio of 4:3 and a density of 96 dpi, a bit depth of 24 bit (8 bit per R, G, B color channel) and a video frame rate of 30 to 60 fps.

The infrared light beam emitter 55 and the infrared light receiver 57 are arranged on a circle (shown in the figure as a stippled line) having a predefined diameter D50 and surrounding the optical axis 62. In this embodiment, the predefined distance d50 is 25 cm with the predefined diameter D50 being smaller than or equal to 5 cm. With this combination of values for the predefined distance d50 and the predefined diameter D50 the emitted infrared light beams can converge on the cervix 63 without becoming obstructed by the vaginal speculum 64 having a typical inner diameter of 1.6 cm and a length of 8 cm (corresponding to a normal depth of the vaginal channel). Of course, the predefined diameter D50 should suitably be larger than 0 cm, for instance, larger than 1 cm or larger than 3 cm. For instance, here, the predefined diameter D50 is 4 cm.

The colposcopy apparatus 50 comprises a user indication unit 60 for providing indications to a user of the colposcopy apparatus 50 to support the user in positioning the colposcopy apparatus 50 based on the currently sensed distance. In particular, in this embodiment, the colposcopy apparatus 50 is adapted to provide an initialization phase during which the infrared light distance sensing unit 55, 57 repeatedly senses the distance to generate a plurality of sensed distances, while a user of the colposcopy apparatus 50 shall move the colposcopy apparatus 50 in substantially a plane in front of the cervix 63, to identify a working distance of the colposcopy apparatus 50 from the cervix 63 based on the plurality of sensed distances, and to store the identified working distance, wherein the indications are further based on the stored working distance. The user indication unit 60, here, is adapted for displaying the acquired image 61 to the user and to visually provide the indications as a visual overlay (not shown in the figure) provided on the displayed image.

In this example, the indications consist of quantitative information giving the deviation of the currently sensed distance from the stored working distance in terms of positive and negative numerical values, such as -1.5 cm or +0.9 cm. Given this information, the user can then be able to more easily tell whether the colposcopy apparatus 50 is aimed at the cervix 63 or not (as described above).

Here, the colposcopy apparatus 50 further comprises an image analysis unit 59 for analysing the acquired image 61 of the cervix 63 to determine information about the focal distance and/or the zoom of the imaging unit 51, wherein the colposcopy apparatus 50 is adapted, during the initialization phase, when the colposcopy apparatus 50 is positioned with respect to the cervix 63 such that the currently sensed distance matches the stored working distance, to adjust the focal distance and the zoom of the imaging unit 51 based on the determined information for the currently acquired image 61 of the cervix 63 and, after adjusting the focal distance and the zoom of the imaging unit 51, to store the currently acquired image 61 of the cervix 63.

The information about the focal distance and the zoom of the imaging unit 51 is determined, here, by image analysis techniques that are able to assess an amount of blur of the currently acquired image 61 and to detect the cervix 63 or a fraction thereof in the currently acquired image 61. Based on the assessed amount of blur of the currently acquired image 61 and the detected cervix 63 or fraction thereof in the currently acquired image 61, the focal distance and the zoom of the imaging unit 51 are then iteratively adjusted. By adjusting the focal distance and the zoom of the imaging unit 51, a sharp image of the cervix 63, which shows the cervix 63 such that it substantially just completely fills the acquired image 61, is acquired and this image 61 is stored as a kind of "gold standard" for later acquired images 61 of the cervix 63.

The adjustment of the focal distance and the zoom of the imaging unit 51, here, comprises a combination of optical and digital techniques. In particular, the optics unit 52 comprises at least one variable-focus lens (not shown in the figure) based, for instance, on liquids, for adjusting the focal distance of the imaging unit 51 and the adjustment of the zoom is performed by means of digital zooming and by the use of different zoom lenses (not shown in the figure).

In this embodiment, the image analysis unit 59 is adapted to determine further information about a difference between the currently acquired image 61 of the cervix 63 and the stored image of the cervix 63, and the colposcopy apparatus 50 is adapted to provide a colposcopy procedure phase during which the focal distance and the zoom of the imaging unit 51 are adjusted based on the determined further information.

By adjusting, during the "actual" colposcopy procedure phase, the focal distance and the zoom of the imaging unit 51 based on the determined further information, sharp image of the cervix 63, which shows the cervix 63 such that it substantially just completely fills the acquired image 61, is acquired even if the colposcopy apparatus 50 is positioned at a distance from the cervix 63 different from the stored working distance. The further information about a difference between the currently acquired image 61 of the cervix 63 and the stored image of the cervix 63 is determined, here, by image analysis techniques that perform a correlation between the currently acquired image 61 of the cervix 63 and the stored image of the cervix 63, wherein the focal distance and/or the zoom of the imaging unit 51 are adjusted to minimize the difference.

While in the second embodiment described with reference to Fig. 5 above, the focal distance and the zoom of the imaging unit 51 are adjusted based a determined further information about a difference between the currently acquired image 61 of the cervix 63 and the stored image of the cervix 63, in other embodiments, the colposcopy apparatus 50 can be adapted to adjust the focal distance and/or the zoom of the imaging unit 51 based on the currently sensed distance. The colposcopy apparatus 50 may comprise a predefined look-up table storing for a range of currently sensed distances of the colposcopy apparatus 50 from the cervix 63 suitable values for adjusting the focal distance and/or the zoom of the imaging unit 51. The range of currently sensed distances may comprise, for instance, distances of the colposcopy apparatus 50 from the cervix ranging from 20 cm to 30 cm. Suitable values for adjusting the focal distance and the zoom of the imaging unit 51 may be calculated as described above.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The present invention relates to a colposcopy apparatus for performing a colposcopy procedure. The colposcopy apparatus comprises an imaging unit comprising an optics unit having an optical axis and an image acquiring unit for acquiring an image of a cervix through the optics unit, and at least three light beam emitting units spaced apart from the optical axis, wherein the at least three light beam emitting units are adapted to emit light beams that are coplanar with the optical axis and that intersect on the optical axis at a predefined distance in front of the colposcopy apparatus, wherein not all of the emitted light beams lie in a same plane.

## Claims

1. A colposcopy apparatus (10) for performing a colposcopy procedure, wherein the colposcopy apparatus (10) comprises:

   - an imaging unit (11) comprising an optics unit (12) having an optical axis (22) and an image acquiring unit (13) for acquiring an image (21) of a cervix (23) through the optics unit (12); and
   - at least three light beam emitting units (14, 15, 16, 17) spaced apart from the optical axis (22), wherein the at least three light beam emitting units (14, 15, 16, 17) are adapted to emit coherent light beams that are coplanar with the optical axis (22) and that intersect on the optical axis (22) at a predefined distance (d10) in front of the colposcopy apparatus (10), wherein not all of the emitted coherent light beams lie in a same plane, and wherein the emitted coherent light beams produce a single observable light spot on the cervix (23) when the colposcopy apparatus (10) is positioned at the predefined distance (d10) from the cervix (23);

   wherein the colposcopy apparatus (10) further comprises:

   an image analysis unit (19) for analysing the acquired image (21) of the cervix (23) with respect to light spots (25, 26, 27, 28) produced by the emitted coherent light beams on the cervical tissue to determine the distance of the colposcopy apparatus (10) from the cervix (23), wherein the colposcopy apparatus (10) is adapted to adjust the focal distance and/or the zoom of the imaging unit (11) based on the determined distance; or
   an image analysis unit (19) for analysing the acquired image (21) of the cervix (23) with respect to light spots (25, 26, 27, 28) produced by the emitted coherent light beams on the cervical tissue to determine information about the positioning of the colposcopy apparatus (10) with respect to the cervix (23); and
   a user indication unit (20) for providing indications to a user of the colposcopy apparatus (10) to support the user in positioning the colposcopy apparatus (10) based on the determined information.

2. The colposcopy apparatus (10) as defined in claim 1, wherein the at least three light beam emitting units (14, 15, 16, 17) comprise four light beam emitting units (14, 15, 16, 17) that are arranged on the top, the left, the bottom, and the right with respect to the acquired image (21).

3. The colposcopy apparatus (10) as defined in claim 1, wherein at least two of the emitted coherent light beams have visually discernible colors.

4. The colposcopy apparatus (10) as defined in claim 1, wherein the at least three light beam emitting units (14, 15, 16, 17) are arranged on a circle having a predefined diameter (D10) and surrounding the optical axis (22), wherein the predefined distance (d10) is preferably between 23 cm and 27 cm with the predefined diameter (D10) being smaller than or equal to 4.6 cm to 5.4 cm, wherein the predefined distance (d10) is more preferably between 24 cm and 26 cm with the predefined diameter (D10) being smaller than or equal to 4.8 cm to 5.2 cm, wherein the predefined distance (d10) is most preferred 25 cm with the predefined diameter (D10) being smaller than or equal to 5 cm.

5. The colposcopy apparatus (10) as defined in claim 1, wherein the indications comprise first indications indicating whether the colposcopy apparatus (10) must be moved towards the cervix (23) or away from the cervix (23) to position the colposcopy apparatus (10) at a distance from the cervix (23) equal to the predefined distance (d10).

6. The colposcopy apparatus (10) as defined in claim 1, wherein the indications comprise second indications indicating whether the colposcopy apparatus (10) is positioned such that the cervix (23) is within the depth of field of the imaging unit (11).

7. The colposcopy apparatus (10) as defined in claim 6, wherein the user indication unit (20) is adapted to display the acquired image (21) to the user, wherein the second indications comprise a visual representation of the depth of field displayed with the acquired image (21).

8. The colposcopy apparatus (10) as defined in claim 1, wherein for changes in the determined distance, the focal distance and/or the zoom of the imaging unit (11) are adjusted such that the size of the cervix (23) or of a fraction thereof shown in the acquired image (21) is kept constant.

9. A colposcopy apparatus (50) for performing a colposcopy procedure, wherein the colposcopy apparatus (50) comprises:

   - an imaging unit (51) comprising an optics unit (52) having an optical axis (62) and an image acquiring unit (53) for acquiring an image (51) of a cervix (63) through the optics unit (52);
   - an infrared light distance sensing unit (55, 57) comprising an infrared light beam emitter (55) and an infrared light receiver (57) spaced apart from the optical axis (62), wherein the infrared light beam emitter (55) is adapted to emit infrared light beams that are coplanar with the optical axis (62) and that intersect on the optical axis (62) at a predefined distance (d50) in front of the colposcopy apparatus (50), wherein the infrared light receiver (57) lies in a same plane with the emitted infrared light beams and the optical axis (62) and is adapted to receive the emitted infrared light beams after they have been reflected, and wherein the infrared light distance sensing unit (55, 57) is adapted to sense a distance based on the emitted and reflected infrared light beams; and
   - a user indication unit (60) for providing indications to a user of the colposcopy apparatus (50) based on the currently sensed distance to support the user in positioning the colposcopy apparatus (50) at a distance from the cervix (63) that is substantially equal to the predefined distance (d50).

10. The colposcopy apparatus (50) as defined in claim 9, wherein the colposcopy apparatus (50) is adapted to provide an initialization phase during which the infrared light distance sensing unit (55, 57) repeatedly senses the distance to generate a plurality of sensed distances, while a user of the colposcopy apparatus (50) shall move the colposcopy apparatus (50) in substantially a plane in front of the cervix (63), to identify a working distance of the colposcopy apparatus (50) from the cervix (63) based on the plurality of sensed distances, and to store the identified working distance, wherein the indications are further based on the stored working distance.

11. The colposcopy apparatus (50) as defined in claim 10, wherein the colposcopy apparatus (50) further comprises:

   - an image analysis unit (59) for analysing the acquired image (61) of the cervix (63) to determine information about the focal distance and/or the zoom of the imaging unit (51),

   wherein the colposcopy apparatus (50) is adapted, during the initialization phase, when the colposcopy apparatus (50) is positioned with respect to the cervix (63) such that the currently sensed distance matches the stored working distance, to adjust the focal distance and/or the zoom of the imaging unit (51) based on the determined information for the currently acquired image (61) of the cervix (63) and, after adjusting the focal distance and/or the zoom of the imaging unit (51), to store the currently acquired image (61) of the cervix (63).

12. The colposcopy apparatus (50) as defined in claim 11, wherein the image analysis unit (59) is adapted to determine further information about a difference between the currently acquired image (61) of the cervix (63) and the stored image of the cervix (63), and wherein the colposcopy apparatus (50) is adapted to provide a colposcopy procedure phase during which the focal distance and/or the zoom of the imaging unit (51) are adjusted based on the determined further information.

13. The colposcopy apparatus (50) as defined in claim 9, the colposcopy apparatus (50) is adapted to adjust the focal distance and/or the zoom of the imaging unit (51) based on the currently sensed distance.

**Patentansprüche**

1. Kolposkopie-Gerät (10) zum Durchführen eines Kolposkopie-Verfahrens, wobei das Kolposkopie-Gerät (10) Folgendes umfasst:

    - eine Bildgebungseinheit (11) mit einer optischen Einheit (12) mit einer optischen Achse (22) und einer Bilderfassungseinheit (13) zum Erfassen eines Bilds (21) eines Gebärmutterhalses (23) mithilfe der optischen Einheit (12); und
    - mindestens drei Lichtstrahl-Emissionseinheiten (14, 15, 16, 17), die einen Abstand zur optischen Achse (22) aufweisen, wobei die mindestens drei Lichtstrahl-Emissionseinheiten (14, 15, 16, 17) kohärente Lichtstrahlen emittieren, die koplanar mit der optischen Achse (22) sind, und die sich auf der optischen Achse (22) in einem vordefinierten Abstand (d10) vor dem Kolposkopie-Gerät (10) schneiden, wobei nicht alle der emittierten kohärenten Lichtstrahlen in einer Ebene liegen, und wobei die emittierten kohärenten Lichtstrahlen einen einzelnen erkennbaren Lichtfleck auf dem Gebärmutterhals (23) erzeugen, wenn das Kolposkopie-Gerät (10) im vordefinierten Abstand (d10) vom Gebärmutterhals (23) positioniert ist;

    wobei das Kolposkopie-Gerät (10) zudem Folgendes umfasst:

    eine Bildanalyseeinheit (19) zum Analysieren des aufgenommenen Bilds (21) des Gebärmutterhalses (23) in Bezug auf Lichtflecken (25, 26, 27, 28), die durch die emittierten kohärenten Lichtstrahlen auf dem Gebärmutterhalsgewebe erzeugt werden, um den Abstand des Kolposkopie-Geräts (10) vom Gebärmutterhals (23) zu ermitteln, wobei das Kolposkopie-Gerät (10) die Brennweite und/oder den Zoom der Bildgebungseinheit (11) anhand des ermittelten Abstands einstellt; oder eine Bildanalyseeinheit (19) zum Analysieren des aufgenommenen Bilds (21) des Gebärmutterhalses (23) in Bezug auf Lichtflecken (25, 26, 27, 28), die durch die emittierten kohärenten Lichtstrahlen auf dem Gebärmutterhalsgewebe erzeugt werden, um Informationen über die Position des Kolposkopie-Geräts (10) in Bezug auf den Gebärmutterhals (23) zu ermitteln; und eine Benutzerhinweis-Einheit (20) zum Bereitstellen von Hinweisen für einen Benutzer des Kolposkopie-Geräts (10), um den Benutzer beim Positionieren des Kolposkopie-Geräts (10) mithilfe der ermittelten Informationen zu unterstützen.

2. Das Kolposkopie-Gerät (10) gemäß Anspruch 1, wobei die mindestens drei Lichtstrahl-Emissionseinheiten (14, 15, 16, 17) vier Lichtstrahl-Emissionseinheiten (14, 15, 16, 17) umfassen, die in Bezug auf das aufgenommene Bild (21) oben, links, unten und rechts angeordnet sind.

3. Das Kolposkopie-Gerät (10) gemäß Anspruch 1, wobei mindestens zwei der emittierten kohärenten Lichtstrahlen über visuell wahrnehmbare Farben verfügen.

4. Das Kolposkopie-Gerät (10) gemäß Anspruch 1, wobei die mindestens drei Lichtstrahlen emittierenden Einheiten (14, 15, 16, 17) auf einem die optische Achse (22) umgebenden Kreis mit einem vordefinierten Durchmesser (D10) angeordnet sind, wobei der vordefinierte Abstand (d10) möglichst zwischen 23 cm und 27 cm liegt, und wobei der vordefinierte Durchmesser (D10) kleiner oder gleich 4,6 cm bis 5,4 cm ist, wobei der vordefinierte Abstand (d10) vorzugsweise zwischen 24 cm und 26 cm liegt, und wobei der vordefinierte Durchmesser (D10) kleiner oder gleich 4,8 cm bis 5,2 cm ist, wobei der vordefinierte Abstand (d10) idealerweise 25 cm beträgt, und wobei der vordefinierte Durchmesser (D10) kleiner oder gleich 5 cm ist.

5. Das Kolposkopie-Gerät (10) gemäß Anspruch 1, wobei die Hinwiese erste Hinweise umfassen, die angeben, ob das Kolposkopie-Gerät (10) zum Gebärmutterhals (23) hin oder vom Gebärmutterhals (23) weg bewegt werden muss, um das Kolposkopie-Gerät (10) in einem Abstand vom Gebärmutterhals (23) zu positionieren, der dem vordefinierten Abstand (d10) entspricht.

6. Das Kolposkopie-Gerät (10) gemäß Anspruch 1, wobei die Hinweise zweite Hinweise umfassen, die angeben, ob das Kolposkopie-Gerät (10) so positioniert ist, dass sich der Gebärmutterhals (23) innerhalb der Tiefenschärfe der Bildgebungseinheit (11) befindet.

7. Das Kolposkopie-Gerät (10) gemäß Anspruch 6, wobei die Benutzerhinweis-Einheit (20) dem Benutzer das aufgenommene Bild (21) anzeigt, wobei die zweiten Hinwiese eine visuelle Darstellung der Tiefenschärfe umfassen, die mit dem erfassten Bild (21) angezeigt wird.

8. Das Kolposkopie-Gerät (10) gemäß Anspruch 1, wobei bei Änderungen des ermittelten Abstands die Brennweite und/oder der Zoom der Bildgebungseinheit (11) so eingestellt werden, dass die Größe des im aufgenommenen Bild (21) dargestellten Gebärmutterhalses (23) oder eines Teils desselben konstant gehalten wird.

9. Ein Kolposkopie-Gerät (50) zum Durchführen eines Kolposkopie-Verfahrens, wobei das Kolposkopie-Gerät (50) Folgendes umfasst:

   - eine Bildgebungseinheit (51) mit einer optischen Einheit (52) mit einer optischen Achse (62) und einer Bilderfassungseinheit (53) zum Erfassen eines Bilds (51) eines Gebärmutterhalses (63) mithilfe der optischen Einheit (52);
   - eine Infrarotlicht-Abstandsensor-Einheit (55, 57), die einen Infrarotlicht-Strahlensender (55) und einen Infrarotlicht-Empfänger (57) umfasst, die einen Abstand zur optischen Achse (62) aufweisen, wobei die Lichtstrahl-Emissionseinheit (55) kohärente Lichtstrahlen emittiert, die koplanar mit der optischen Achse (62) sind, und die sich auf der optischen Achse (62) in einem vordefinierten Abstand (d50) vor dem Kolposkopie-Gerät (50) schneiden, wobei der Infrarotlicht-Empfänger (57) in derselben Ebene wie die emittierten Infrarotlichtstrahlen und die optische Achse (62) liegt und die emittierten Infrarotlichtstrahlen empfängt, nachdem diese reflektiert wurden, und wobei die Infrarotlicht-Abstandsensor-Einheit (55, 57) den Abstand beruhend auf den emittierten und reflektierten Infrarotlichtstrahlen erfasst; und
   - eine Benutzerhinweis-Einheit (60) zum Bereitstellen von Hinweisen für einen Benutzer des Kolposkopie-Geräts (50) auf Grundlage des aktuell erfassten Abstands, um den Benutzer beim Positionieren des Kolposkopie-Geräts (50) in einem Abstand vom Gebärmutterhals (63) zu unterstützen, der im Wesentlichen dem vordefinierten Abstand (d50) entspricht.

10. Das Kolposkopie-Gerät (50) gemäß Anspruch 9, wobei das Kolposkopie-Gerät (50) eine Initialisierungsphase bereitstellt, während der die Infrarotlicht-Abstandsensor-Einheit (55, 57) wiederholt den Abstand erfasst, um mehrere erfasste Abstände zu generieren, während ein Benutzer des Kolposkopie-Geräts (50) das Kolposkopie-Gerät (50) im Wesentlichen in einer Ebene vor dem Gebärmutterhals (63) bewegt, um anhand der mehreren erfassten Abstände einen Arbeitsabstand des Kolposkopie-Geräts (50) zum Gebärmutterhals (63) zu ermitteln und den ermittelten Arbeitsabstand zu speichern, wobei die Hinweise zudem auf dem gespeicherten Arbeitsabstand beruhen.

11. Das Kolposkopie-Gerät (50) gemäß Anspruch 10, wobei das Kolposkopie-Gerät (50) zudem Folgendes umfasst:

    - eine Bildanalyseeinheit (59) zum Analysieren des erfassten Bilds (61) des Gebärmutterhalses (63), um Informationen über die Brennweite und/oder den Zoom der Bildgebungseinheit (51) zu ermitteln,

    wobei das Kolposkopie-Gerät (50) in der Initialisierungsphase, in der das Kolposkopie-Gerät (50) in Bezug auf den Gebärmutterhals (63) so positioniert ist, dass der aktuell erfasste Abstand mit dem gespeicherten Arbeitsabstand übereinstimmt, die Brennweite und/oder den Zoom der Bildgebungseinheit (51) beruhend auf den ermittelten Informationen für das aktuell erfasste Bild (61) des Gebärmutterhalses (63) einstellt und nach dem Einstellen der Brennweite und/oder des Zooms der Bildgebungseinheit (51) das aktuell erfasste Bild (61) des Gebärmutterhalses (63) speichert.

12. Das Kolposkopie-Gerät (50) gemäß Anspruch 11, wobei die Bildanalyseeinheit (59) weitere Informationen über den Unterschied zwischen dem aktuell erfassten Bild (61) des Gebärmutterhalses (63) und dem gespeicherten Bild des Gebärmutterhalses (63) ermittelt, und wobei das Kolposkopie-Gerät (50) eine Phase für das Kolposkopie-Verfahren bereitstellt, während der die Brennweite und/oder der Zoom der Bildgebungseinheit (51) anhand der ermittelten weiteren Informationen eingestellt werden.

13. Das Kolposkopie-Gerät (50) gemäß Anspruch 9, wobei das Kolposkopie-Gerät (50) die Brennweite und/oder den Zoom der Bildgebungseinheit (51) anhand des aktuell erfassten Abstands einstellt.

**Revendications**

1. Appareil de colposcopie (10) destiné à la réalisation d'une procédure de colposcopie, dans lequel l'appareil de colposcopie (10) comprend :

   - une unité d'imagerie (11) comprenant une unité optique (12) présentant un axe optique (22) et une unité d'acquisition d'image (13) destinée à l'acquisition d'une image (21) d'un col utérin (23) à travers l'unité optique (12) ; et
   - au moins trois unités émettrices (14, 15, 16, 17) de faisceau lumineux espacées de l'axe optique (22), dans lequel les au moins trois unités d'émission (14, 15, 16, 17) de faisceau lumineux sont conçues pour émettre des faisceaux lumineux cohérents, lesquels sont coplanaires avec l'axe optique (22) et lesquels se coupent sur l'axe optique (22) à une distance prédéfinie (d10) devant l'appareil de colposcopie (10), dans lequel tous les faisceaux lumineux cohérents émis ne se trouvent pas dans un même plan, et dans lequel les faisceaux lumineux cohérents émis produisent un seul point lumineux observable sur le col utérin (23) lorsque l'appareil de colposcopie (10) est positionné à la distance prédéfinie (d10) du col utérin (23) ;

   dans lequel l'appareil de colposcopie (10) comprend en outre :

   une unité d'analyse d'image (19) destinée à l'analyse de l'image (21) acquise du col utérin (23) par rapport aux points lumineux (25, 26, 27, 28) produits par les faisceaux lumineux cohérents émis sur le tissu cervical pour déterminer la distance de l'appareil de colposcopie (10) à partir du col utérin (23), dans lequel l'appareil de colposcopie (10) est conçu pour ajuster la distance focale et/ou la distance focale variable de l'unité d'imagerie (11) en fonction de la distance déterminée ; ou
   une unité d'analyse d'image (19) destinée à l'analyse de l'image (21) acquise du col utérin (23) par rapport aux points lumineux (25, 26, 27, 28) produits par les faisceaux lumineux cohérents émis sur le tissu cervical pour déterminer des informations sur le positionnement de l'appareil de colposcopie (10) par rapport au col utérin (23) ; et
   une unité d'indication d'utilisateur (20) destinée à la fourniture des indications à un utilisateur de l'appareil de colposcopie (10) pour aider l'utilisateur à positionner l'appareil de colposcopie (10) en fonction des informations déterminées.

2. Appareil de colposcopie (10) selon la revendication 1, dans lequel les au moins trois unités émettrices (14, 15, 16, 17) de faisceau lumineux comprennent quatre unités émettrices (14, 15, 16, 17) de faisceau lumineux, lesquelles sont disposées en haut, à gauche, en bas et à droite par rapport à l'image (21) acquise.

3. Appareil de colposcopie (10) selon la revendication 1, dans lequel les au moins deux des faisceaux lumineux cohérents émis présentent des couleurs visuellement discernables.

4. Appareil de colposcopie (10) selon la revendication 1, dans lequel les au moins trois unités émettrices (14, 15, 16, 17) de faisceau lumineux sont disposées sur un cercle présentant un diamètre prédéfini (D10) et entourant l'axe optique (22), dans lequel la distance prédéfinie (d10) est de préférence comprise entre 23 cm et 27 cm, le diamètre prédéfini (D10) étant inférieur ou égal à 4,6 cm à 5,4 cm, dans lequel la distance prédéfinie (d10) est de préférence encore comprise entre 24 cm et 26 cm, le diamètre prédéfini (D10) étant inférieur ou égal à 4,8 cm à 5,2 cm, dans lequel la distance prédéfinie (d10) est de préférence de 25 cm, le diamètre prédéfini (D10) étant inférieur ou égal à 5 cm.

5. Appareil de colposcopie (10) selon la revendication 1, dans lequel les indications comprennent des premières indications indiquant si l'appareil de colposcopie (10) doit être déplacé vers le col utérin (23) ou éloigné du col utérin (23) pour positionner l'appareil de colposcopie (10) à une distance du col utérin (23) égale à la distance prédéfinie (d10).

6. Appareil de colposcopie (10) selon la revendication 1, dans lequel les indications comprennent des secondes indications indiquant si l'appareil de colposcopie (10) est positionné de telle sorte que le col utérin (23) se trouve dans la profondeur de champ de l'unité d'imagerie (11).

7. Appareil de colposcopie (10) selon la revendication 6, dans lequel l'unité d'indication d'utilisateur (20) est conçue pour afficher l'image (21) acquise à l'utilisateur, dans lequel les secondes indications comprennent une représentation visuelle de la profondeur de champ affichée avec l'image (21) acquise.

8. Appareil de colposcopie (10) selon la revendication 1, dans lequel pour les changements de la distance déterminée, la distance focale et/ou la distance focale variable de l'unité d'imagerie (11) sont ajustés de telle sorte que la taille du col utérin (23) ou d'une fraction de celui-ci montré dans l'image (21) acquise est conservée constant.

9. Appareil de colposcopie (50) destiné à la réalisation d'une procédure de colposcopie, dans lequel l'appareil de colposcopie (50) comprend :

- une unité d'imagerie (51) comprenant une unité optique (52) présentant un axe optique (62) et une unité d'acquisition d'image (53) destinée à l'acquisition d'une image (51) d'un col utérin (63) à travers l'unité optique (52) ;
- une unité de détection (55, 57) de distance à lumière infrarouge comprenant un émetteur (55) de faisceau de lumière infrarouge et un récepteur (57) de lumière infrarouge espacés de l'axe optique (62), dans lequel l'émetteur (55) de faisceau lumineux infrarouge est conçu pour émettre des faisceaux lumineux infrarouges, lesquels sont coplanaires avec l'axe optique (62) et lesquels se coupent sur l'axe optique (62) à une distance prédéfinie (d50) devant l'appareil de colposcopie (50), dans lequel le récepteur (57) de lumière infrarouge se situe dans un même plan avec les faisceaux lumineux infrarouges émis et l'axe optique (62) et est conçu pour recevoir les faisceaux lumineux infrarouges émis après qu'ils ont été réfléchis, et dans lequel l'unité de détection (55, 57) de distance à lumière infrarouge est conçue pour détecter une distance en fonction des faisceaux lumineux infrarouge émis et réfléchis ; et

une unité d'indication d'utilisateur (60) destinée à

- la fourniture des indications à un utilisateur de l'appareil de colposcopie (50) en fonction de la distance actuellement détectée pour aider l'utilisateur à positionner l'appareil de colposcopie (50) à une distance du col utérin (63), laquelle est sensiblement égale à la distance prédéfinie (d50).

10. Appareil de colposcopie (50) selon la revendication 9, dans lequel l'appareil de colposcopie (50) est conçu pour fournir une phase d'initialisation au cours de laquelle l'unité de détection de distance à lumière infrarouge (55, 57) détecte à plusieurs reprises la distance pour générer une pluralité de distances détectées, un utilisateur de l'appareil de colposcopie (50) devant déplacer l'appareil de colposcopie (50) dans sensiblement un plan devant le col utérin (63), pour identifier une distance de travail de l'appareil de colposcopie (50) à partir du col utérin (63) en fonction de la pluralité de distances détectées, et pour mémoriser la distance de travail identifiée, les indications étant en outre basées sur la distance de travail mémorisée.

11. Appareil de colposcopie (50) selon la revendication 10, dans lequel l'appareil de colposcopie (50) comprend en outre :

- une unité d'analyse d'image (59) destinée à l'analyse de l'image (61) acquise du col utérin (63) pour déterminer des informations sur la distance focale et/ou la distance focale variable de l'unité d'imagerie (51),

dans lequel l'appareil de colposcopie (50) est conçu, lors de la phase d'initialisation, lorsque l'appareil de colposcopie (50) est positionné par rapport au col utérin (63) de telle sorte que la distance actuellement détectée correspond à la distance de travail stockée, pour ajuster la distance focale et /ou la distance focal variable de l'unité d'imagerie (51) en fonction des informations déterminées pour l'image (61) actuellement acquise du col utérin (63) et, après réglage de la distance focale et/ou de la distance focale variable de l'unité d'imagerie (51), pour mémoriser l'image (61) actuellement acquise du col utérin (63).

12. Appareil de colposcopie (50) selon la revendication 11, dans lequel l'unité d'analyse d'image (59) est conçue pour déterminer d'autres informations concernant une différence entre l'image (61) actuellement acquise du col utérin (63) et l'image mémorisée du col utérin (63), et dans lequel l'appareil de colposcopie (50) est conçu pour fournir une phase de procédure de colposcopie lors de laquelle la distance focale et/ou la distance focale variable de l'unité d'imagerie (51) sont ajustées en fonction des informations supplémentaires déterminées.

13. Appareil de colposcopie (50) selon la revendication 9, l'appareil de colposcopie (50) étant conçu pour ajuster la distance focale et/ou la distance focale variable de l'unité d'imagerie (51) en fonction de la distance actuellement détectée.

FIG. 1

FIG. 2

FIG. 3

FIG. 4

EP 3 142 540 B1

FIG. 5

EP 3 142 540 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060215406 A
- WO 2014006549 A
- US 20100025566 A
- US 20120249764 A
- US 6277067 B1

**Non-patent literature cited in the description**

- **MATUNGKA R. et al.** Image Registration Using Adaptive Polar Transform. *IEEE Transactions on Inage Processing,* October 2009, vol. 18 (10), 2340-2354